# FASCICULE DE BREVET EUROPEEN

(11) **EP 0 600 806 B1**
(45) Date de publication et mention de la délivrance du brevet: **04.06.1997**
(21) Numéro de dépôt: 93420467.8
(22) Date de dépôt: 25.11.1993
(51) Int. Cl.: A61F 2/38

(54) **Prothèse tricompartimentale du genou**
Aus drei Teilabschnitten gebildete Knieprothese
Tricompartimental knee prosthesis

(30) Priorité: 01.12.1992 FR 9214726
(43) Date de publication de la demande: 08.06.1994
(73) Titulaire: MEDINOV SA, F-42312 Roanne Cedex (FR)
(72) Inventeur: Colombier, Michel, 42300 Roanne (FR); Delfosse, Jacques Dr., 54000 Nancy (FR); Moati, Jean Claude Dr., 92130 Issy Les Moulineaux (FR); Chatelet, Jean Christophe, Dr., F-69007 Lyon (FR); Ferreira, André Dr., 69300 Caluire (FR); Brugere, Pierre Dr., 51300 Vitry le Francois (FR); Charpenet, Rémy Dr., 88150 Thaon les Vosges (FR); Paulin, Marc Dr., 52000 Chaumont (FR); Preaut, Jacques Dr., 55000 Bar le Duc (FR); Khenifar, Brahim Dr., 58170 Luzy (FR); Huc de Bat, Jean Michel Dr., 13540 Puyricard (FR)
(74) Mandataire: Dupuis, François

(56) Documents cités:
- EP-A- 0 327 495
- FR-A- 2 521 421
- US-A- 4 081 866
- R. S. LASKIN 'total knee replacement' 1992 , SPRINGER-VERLAG , LONDON chapitre 10, pages 161 à 182 * page 173, colonne de droite, alinéa 2 - page 174, colonne de gauche, alinéa 1; figure 10.24 *
- R. S. LASKIN 'total knee replacement' 1992 , SPRINGER-VERLAG , LONDON chapitre 7, pages 85 à 111 * page 90, colonne de droite, ligne 2 - page 92, colonne de gauche, ligne 1 *

## Description

L'invention concerne plus particulièrement, une prothèse totale du genou.

D'une manière parfaitement connue pour un homme du métier, ce type de prothèse comprend un implant fémoral et un implant tibial. L'implant fémoral présente une échancrure délimitant deux patins condyliens d'appui et de glissement réunis par une trochlée. Sur cette trochlée, peut prendre appui un composant rotulien. Les patins condyliens sont en appui sur l'implant tibial par l'intermédiaire d'une embase ou plateau tibial, le plus souvent en polyéthylène.

Généralement, la conception d'une prothèse du genou tricompartimentale est basée sur une approche synthètique de l'articulation anatomique du genou. Quelle que soit la conception de ce type de prothèse, les formes et profils tendent à précontraindre l'articulation du genou. L'implant prothétique impose donc une cinématique réelle différente de la théorie.

Pour tenter de remédier à ces inconvénients, on a proposé un ensemble prothétique pour l'articulation du genou, comme il ressort de l'enseignement du brevet FR 2589720. Ce brevet définit une prothèse trochléo-bicondiylienne monobloc dont les formes et dimensions sont déterminées pour s'adapter à l'anatomie du genou, notamment du fémur qui est considéré selon un échantillon représentatif.

Là encore, cette conception relève d'une approche synthétique de l'articulation du genou.

Le problème que se propose de résoudre l'invention est de concevoir une prothèse sans contrainte afin de diminuer les risques d'usure et de descellement des différents éléments constitutifs.

Pour résoudre ce problème il a donc été conçu et mis au point une prothèse qui résulte d'une étude cinématique morphométrique du genou afin de définir la forme des éléments fonctionnels.

Selon l'invention, la prothèse tricompartimentale du genou est remarquable en ce que l'implant fémoral a une forme résultant de la combinaison des caractéristiques suivantes :
- les patins condyliens interne et externe ont des rayons de courbure différents dans le plan sagittal,
- les surfaces d'appui et de glissement des patins condyliens interne et externe ont une largeur et une section différentes dans le plan frontal,
- les patins condyliens interne et externe ont des amplitudes de roulement différentes en partie postérieure,
- la trochlée délimite une surface d'une section d'un tore géométrique dans les plans frontal et sagittal en étant relevée d'une manière anatomique du côté externe,
- l'empattement des surfaces d'appui et de glissement des patins condyliens est augmenté par rapport à l'anatomie,
- les patins condyliens sont aplatis pour favoriser la répartition des pressions.

Pour résoudre le problème posé de tenir compte du mouvement de rotation vers l'extérieur de la jambe au moment de la flexion, le rayon de courbure du patin condylien interne est inférieur au rayon de courbure du patin condylien externe.

Ces dispositions permettent de diminuer l'usure entre les patins condyliens et le plateau tibial, en opposition aux prothèses connues où les patins condyliens présentent la même courbure, ce qui augmente le glissement relatif.

Pour tenir compte du fait que le condyle interne présente une surface d'appui et de glissement plus étroite, malgré le fait qu'il soit le plus chargé à partir de l'analyse des forces sur le plan statique, le patin condylien interne présente transversalement une surface d'appui et de glissement plus étroite et aplatie que celle du patin condylien externe.

Un autre problème que se propose de résoudre l'invention est de traiter les infections dégénératives et destructrices du compartiment interne du genou.

Pour le respect de l'anatomie du condyle interne, les bords latéraux de la partie antérieure du patin condylien interne présentent un rayon de courbure correspondant à l'oblicuité du condyle interne osseux dans le plan horizontal.

Pour améliorer la flexion, la longueur de la courbure postérieure est plus grande sur les patins condyliens, de sorte que leur épaisseur, au niveau de cette partie postérieure, est plus importante que celle connue à ce jour sur les prothèses existantes.

Pour résoudre le problème posé de diminuer les risques d'erreur de positionnement de l'échancrure trochléenne par rapport à la rotule, et obtenir un effet auto-stabilisant et auto-centrant dans le plan frontal, la surface torique de la trochlée est déterminée pour que dans le plan sagittal, le cercle équatorial du tore de la trochlée respecte approximativement la courbure anatomique, la section du tore étant décalée par rapport à l'axe principal d'un angle (α) du côté interne et d'un angle (2α) du côté externe.

Il apparait donc que la trochlée résulte d'une surface mathématique dans le plan frontal et dans le plan sagittal. Ces dispositions permettent d'éviter en outre, les luxations externes de la rotule provoquées par les forces résultantes externes dans le mouvement de flexion.

Suivant une autre caractéristique, la distance entre les surfaces d'appui et de glissement des patins condyliens est augmentée par rapport à l'anatomie du genou, pour décaler vers l'extérieur, les parties en contact avec l'élément tibial.

Compte-tenu du problème posé d'avoir un appui péricortical, l'implant tibial présente une embase asymétrique avec une longueur antéro-postérieure plus importante du côté externe.

L'invention est exposée, ci-après plus en détail à l'aide des dessins annexés, dans lesquels :

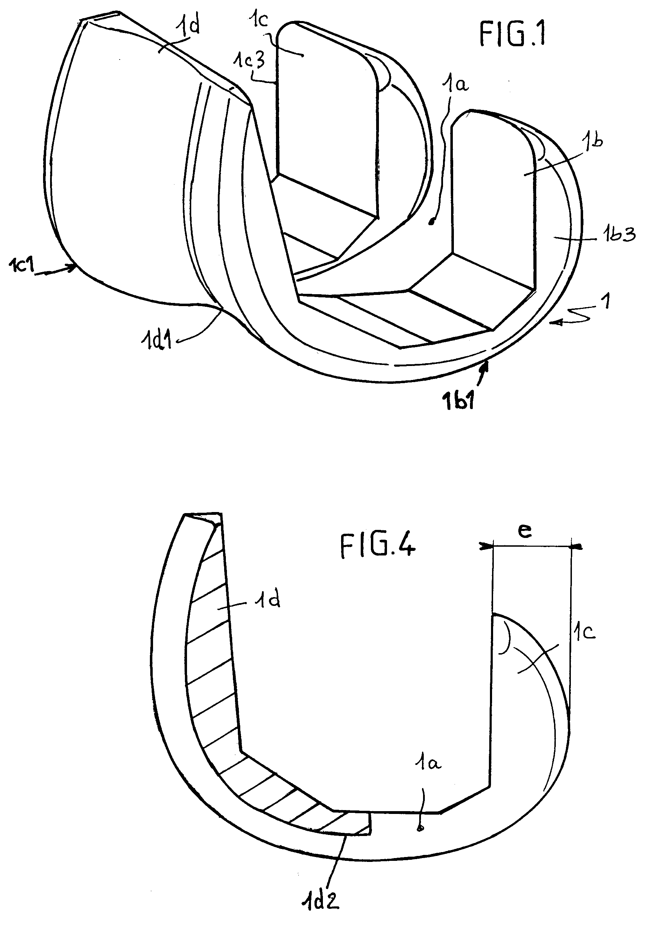

La figure 1 est une vue en perspective de l'implant fémoral de la prothèse.
La figure 2 est une vue de face considérée du côté postérieur de l'implant fémoral.
La figure 3 est une vue en plan correspondant à la figure 2.
La figure 4 est une vue en coupe transversale considérée selon la ligne 4.4 de la figure 2.
La figure 5 est une vue de face de la prothèse tricompartimentale du genou.

D'une manière connue l'implant fémoral (1) présente une échancrure (1a) délimitant deux patins d'appui condyliens (1b) (1c) réunis par une trochlée (1d). Les différents éléments fonctionnels de l'implant (1), ont des formes et profils résultant d'une étude cinématique morphométrique du genou.

Dans ce but, les patins condyliens internes (1b) et externes (1c) ont des rayons de courbure (1b1) (1c1) différents dans le plan sagittal pour améliorer la flexion. Compte-tenu du mouvement de rotation vers l'extérieur de la jambe au moment de la flexion, le rayon de courbure (1b1) du patin condylien interne (1b) est inférieur au rayon de courbure du patin condylien externe.

A noter que les patins condyliens (1b) et (1c) sont divergents, toujours dans le but d'améliorer la flexion.

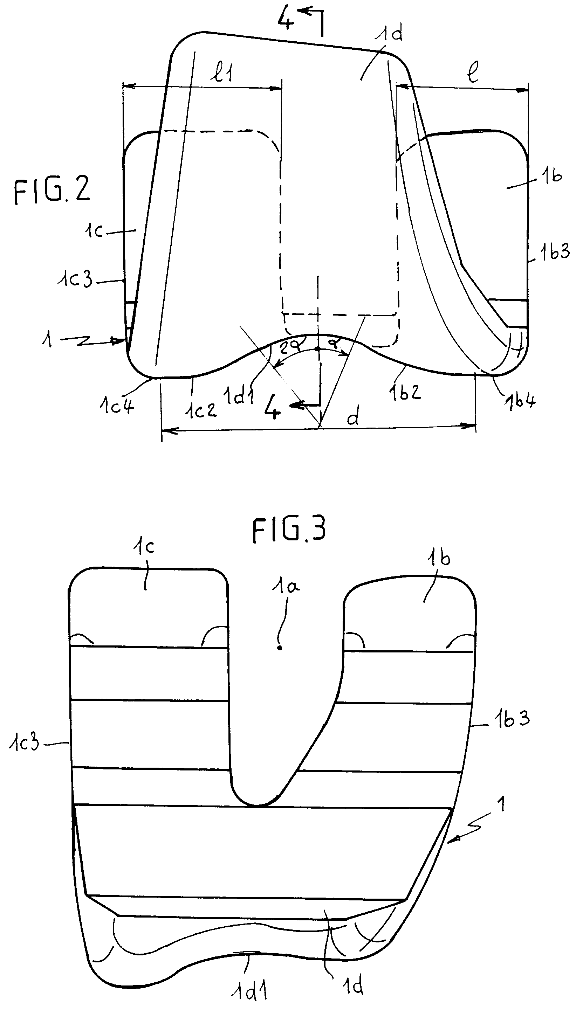

Les surfaces d'appui et de glissement (1b2) (1c2) des patins condyliens interne (1b) et externe (1c) ont une largeur et une forme différente. Comme le montre notamment la figure 2, la surface d'appui du patin condylien interne (1b) présente une largeur (l) inférieure à la largeur (l1) de la surface de glissement du patin condylien externe (1c). De même, les patins condyliens interne et externe présentent transversalement les surfaces d'appui (1b2) et (1c2) aplaties.

A noter que les bords latéraux de la partie antérieure du patin condylien interne (1b) présentent un rayon de courbure (1b3) correspondant à l'oblicuité du condyle interne osseux dans le plan horizontal.

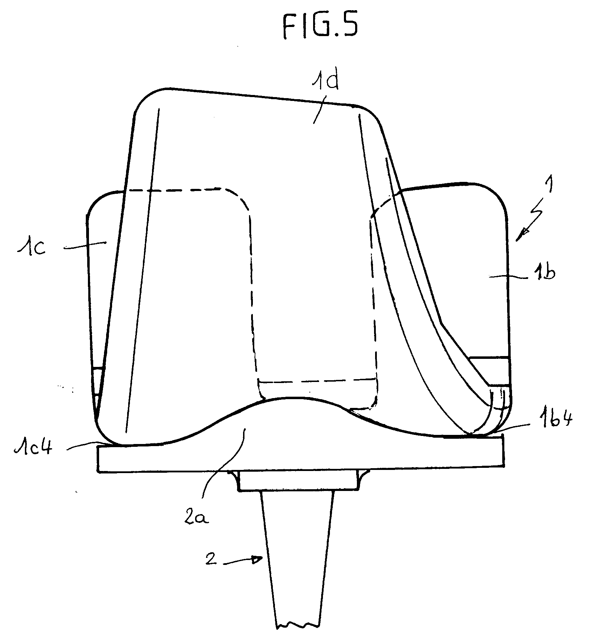

Etant donné que le condyle externe en flexion maximum du genou parcourt une distance supérieure à celle parcourue par le condyle interne, la longueur de la courbure postérieure (1c3) du patin condylien externe est supérieure à celle du patin condylien interne (1b). Les épaisseurs (e) des patins condylien (1b) et (1c), au niveau de la partie postérieure de l'implant, sont plus importantes qu'habituellement (figure 4). Cet artifice permet d'augmenter la longueur de la génératrice du condyle, ce qui permet d'augmenter l'angle de flexion.

La distance (d) entre les surfaces d'appui et de glissement (1b2) (1c2) des patins condyliens (1b) et (1c) est augmentée par rapport à l'anatomie. Cette augmentation a pour effet de décaler vers l'extérieur, les parties en contact (1b4) (1c4) avec l'élément tibial.

Suivant une autre caractéristique, la trochlée (1d) délimite une surface torique (1d1) dans le plan frontal et dans le plan sagittal (1d2). En outre, la section du tore est décalée angulairement pour éviter les luxations externes de la rotule dûes aux forces résultantes extérieures dans le mouvement de flexion. A cet effet, la section de la surface torique (1d1) est décalée par rapport à l'axe principale de la prothèse, d'un angle (α) du côté interne et d'un angle (2α) du côté externe (figure 2).

Pour permettre un appui pericortical, l'embase (2a) de l'implant tibial (2) est asymétrique en présentant un compartiment externe de longueur antéro-postérieure supérieure à celle du compartiment interne. De même, pour supprimer toute contrainte induite en flexion, les plateaux tibiaux sont plats avec un bord postérieur anti-luxation.

Les avantages ressortent bien de la description, en particulier on souligne et on rappelle la forme des éléments fonctionnels de la prothèse, notamment de l'implant fémoral résultant d'une étude cinématique et morphométrique du genou permettant de concevoir une prothèse sans contrainte, afin de diminuer les risques d'usure et/ou de descellement.

## Revendications

1. Prothèse tricompartimentale du genou comprenant un implant fémoral (1) et un implant tibial (2), l'implant fémoral présentant une échancrure délimitant deux patins condyliens d'appui (1b) (1c) divergents et réunis par une trochlée (1d), caractérisée en ce que l'implant fémoral a une forme résultant de la combinaison des caractéristiques suivantes :
- les patins condyliens interne (1b) et externe (1c) ont des rayons de courbure différents dans le plan sagittal,
- les surfaces d'appui et de glissement (1b2) (1c2) des patins condyliens interne (1b) et externe (1c) ont une largeur et une section différentes dans le plan frontal,
- les patins condyliens interne (1b) et externe (1c) ont des amplitudes de roulement différentes en partie postérieure,
- la trochlée (1d) délimite une surface d'une section d'un tore (1d1) géométrique dans les plans frontal et sagittal en étant relevée d'une manière anatomique du côté externe,
- l'empattement des surfaces d'appui et de glissement des patins condyliens est augmenté par rapport à l'anatomie,
- les patins condyliens sont aplatis pour favoriser la répartition des pressions.

2. Prothèse selon la revendication 1, caractérisée en ce que le rayon de courbure du patin condylien interne (1b) est inférieur au rayon de courbure du patin condylien externe (1c).

3. Prothèse selon l'une quelconque des revendications 1 et 2, caractérisée en ce que les bords latéraux (1b3) de la partie antérieure du patin condylien interne (1b) présentent un rayon de courbure correspondant à l'oblicuité du condyle interne osseux dans le plan horizontal.

4. Prothèse selon la revendication 1, caractérisée en ce que le patin condylien interne (1b) présente transversalement une surface d'appui et de glissement (1b2) plus étroite que celle du patin condylien externe (1c).

5. Prothèse selon la revendication 1, caractérisée en ce les patins condyliens interne (1b) et externe (1c) présentent transversalement des surfaces d'appui aplaties.

6. Prothèse selon la revendication 1, caractérisée en ce que la longueur de la courbure postérieure (1c3) du patin condylien externe (1c) est supérieure à celle du patin condylien interne (1b), les épaisseurs des patins condyliens (1b) et (1c), au niveau de la partie postérieure étant augmentées.

7. Prothèse selon la revendication 1, caractérisée en ce que dans le plan sagittal et dans le plan frontal, la trochlée (1d) est en forme de tore, dont la section est décalée par rapport à l'axe principal d'un angle (α) du côté interne et, très sensiblement, d'un angle (2α) du côté externe.

8. Prothèse selon la revendication 1, caractérisée en ce que la distance entre les surfaces d'appui et de glissement (1b2) (1c2) des patins condyliens est augmenté par rapport à l'anatomie du genou, pour décaler vers l'extérieur, les parties en contact avec l'élément tibial.

9. Prothèse selon la revendication 1, caractérisée en ce que l'implant tibial (2) présente une embase asymétrique (2a) avec une longueur antéropostérieure plus importante du côté externe.

10. Prothèse selon la revendication 9, caractérisée en ce que l'embase tibiale est plate avec un bord postérieur anti luxation.

## Claims

1. Three-compartment knee prosthesis comprising a femoral implant (1) and a tibial implant (2), the femoral implant having a cutout which delimits two divergent condylar support pads (1b) (1c) joined by a trochlea (1d), characterised in that the shape of the femoral implant is a result of a combination of the following characteristics:
- the internal (1b) and external (1c) condylar pads have different radii of curvature in the sagittal plane,
- the bearing and wearing surfaces (1b2) (1c2) of the internal (1b) and external (1c) condylar pads are of different width and cross section in the frontal plane,
- the internal (1b) and external (1c) condylar pads have different rolling amplitudes in the posterior part,
- the trochlea (1d) delimits a surface having a geometrical toroidal section (1d1) in the frontal and sagittal planes and is raised in an anatomical manner on its outer side,
- the spread of the bearing and wearing surfaces of the condylar pads is increased in relation to the anatomy,
- the condylar pads are flattened to improve the distribution of pressure.

2. Prosthesis as claimed in claim 1, characterised in that the radius of curvature of the internal condylar pad (1b) is less than the radius of curvature of the external condylar pad (1c).

3. Prosthesis as claimed in claims 1 and 2, characterised in that the lateral edges (1b3) of the anterior part of the internal condylar pad (1b) has a radius of curvature that corresponds to the obliquity of the bony internal condyle in the horizontal plane.

4. Prosthesis as claimed in claim 1, characterised in that the internal condylar pad (1b) has a transverse bearing and wearing surface (1b2) that is narrower than that of the external condylar pad (1c).

5. Prosthesis as claimed in claim 1, characterised in that the internal (1b) and external (1c) condylar pads have flattened transverse bearing surfaces.

6. Prosthesis as claimed in claim 1, characterised in that the length of the posterior curve (1c3) of the external condylar pad (1c) exceeds that of the internal condylar pad (1b) with the thicknesses of the condylar pads (1b) and (1c) being increased at the level of the posterior part.

7. Prosthesis as claimed in claim 1, characterised in that, in the sagittal plane and in the frontal plane, the trochlea (1d) is in the shape of a torus of which the cross section is offset relative to the main axis by an angle (α) on the inner side and, essentially, by an angle (2α) on the outer side.

8. Prosthesis as claimed in claim 1, characterised in that the distance between the bearing and wearing surfaces (1b2) (1c2) of the condylar pads is increased relative to the anatomy of the knee in order to offset those parts that are in contact with the tibial element towards the outside.

9. Prosthesis as claimed in claim 1, characterised in that the tibial implant (2) has an asymmetric base (2a) having an anterior posterior length that is greater on the outer side.

10. Prosthesis as claimed in claim 9, characterised in that the tibial base is flat and has a posterior anti-luxation edge.

## Patentansprüche

1. Dreiteilige Knieprothese mit einem femoralen (1) und einem tibialen (2) Implantat, wobei das femorale Implantat eine Einkerbung aufweist, durch die zwei auseinanderlaufende und durch eine Trochlea (1d) verbundene Kondylenauflageschuhe (1b) (1c) umschrieben werden, dadurch gekennzeichnet, daß das femorale Implantat eine Form besitzt, die sich aus der Kombination der folgenden Merkmale ergibt:
- der innere (1b) und der äußere (1c) Kondylenschuh haben in sagittaler Ebene unterschiedliche Krümmungsradien,
- die Auflage- und Gleitflächen (1b2) (1c2) des inneren (1b) und äußeren (1c) Kondylenschuhs weisen in frontaler Ebene Unterschiede in Breite und Querschnitt auf,
- der innere (1b) und äußere (1c) Kondylenschuh besitzen im hinteren Teil unterschiedliche Rollamplituden,
- die Trochlea (1d) begrenzt in frontaler und sagittaler Ebene eine Fläche mit dem Querschnitt eines geometrischen Torus (1d1), wobei sie auf der Außenseite in anatomischer Weise angehoben ist,
- die Spanne der Auflage- und Gleitflächen der Kondylenschuhe ist im Vergleich zu den anatomischen Verhältnissen erhöht,
- die Kondylenschuhe sind abgeflacht, um die Druckverteilung zu begünstigen.

2. Prothese nach Anspruch 1, dadurch gekennzeichnet, daß der Krümmungsradius des inneren Kondylenschuhs (1b) geringer ist als der Krümmungsradius des äußeren Kondylenschuhs (1c).

3. Prothese nach Anspruch 1 oder 2, dadurch gekennzeichnet, daß die Seitenränder (1b3) des vorderen Teils des inneren Kondylenschuhs (1b) einen Krümmungsradius aufweisen, der der Schräge des inneren Knochenkondylus in horizontaler Ebene entspricht.

4. Prothese nach Anspruch 1, dadurch gekennzeichnet, daß der innere Kondylenschuh (1b) in Querrichtung eine schmalere Auflage- und Gleitfläche (1b2) aufweist als diejenige des äußeren Kondylenschuhs (1c).

5. Prothese nach Anspruch 1, dadurch gekennzeichnet, daß der innere (1b) und äußere (1c) Kondylenschuh in Querrichtung abgeflachte Auflageflächen aufweisen.

6. Prothese nach Anspruch 1, dadurch gekennzeichnet, daß die hintere Krümmung (1c3) des äußeren Kondylenschuhs (1c) länger ist als diejenige des inneren Kondylenschuhs (1b), wodurch die Kondylenschuhe (1b) und (1c) im Bereich des hinteren Teils dicker sind.

7. Prothese nach Anspruch 1, dadurch gekennzeichnet, daß die Trochlea (1d) in sagittaler und frontaler Ebene eine torische Form aufweist, deren Querschnitt gegenüber der Hauptachse eines Winkels (α) auf der Innenseite und ziemlich genau eines Winkels (2α) auf der Außenseite versetzt ist.

8. Prothese nach Anspruch 1, dadurch gekennzeichnet, daß der Abstand zwischen den Auflage- und Gleitflächen (1b2) (1c2) der Kondylenschuhe im Verhältnis zur Knieanatomie erhöht ist, um die mit dem tibialen Element in Kontakt stehenden Teile nach außen zu versetzen.

9. Prothese nach Anspruch 1, dadurch gekennzeichnet, daß das das Tibiaimplantat (2) eine asymmetrische Basis (2a) aufweist, deren anterior-posteriore Länge auf der Außenseite größer ist.

10. Prothese nach Anspruch 9, dadurch gekennzeichnet, daß die Tibiabasis flach ist und zur Verhinderung von Ausrenkungen einen hinteren Rand besitzt.
